# EUROPEAN PATENT APPLICATION

(11) **EP 1 541 195 A1**
(43) Date of publication of application: **15.06.2005**
(21) Application number: 04078361.5
(22) Date of filing: 10.12.2004
(51) Int. Cl.: A61N 5/10

(54) **Radiation treatment system utilizing therapeutic agent and associated identifier**

(30) Priority: 12.12.2003 US 529124 P; 13.10.2004 US 965434
(71) Applicant: Siemens Medical Solutions USA, Inc., Malvern, PA 19355-1406 (US)
(72) Inventor: Moore, Terrence E., 94523 Pleasant Hill, CA (US); Svatos, Michelle Marie, 94602 Oakland, CA (US); Weil, Michael D., 80524 Port Collins, CO (US)
(74) Representative: Condon, Neil

(57) **Abstract**

A system may include a radiation treatment agent (10) to treat tissue in response to received X-ray radiation and an identifier (15) associated with the radiation treatment agent. The identifier may be usable to identify a radiation treatment plan (212). In some embodiments, a radiation treatment plan associated with a patient is generated (S401), the radiation treatment plan is associated with an identifier and a patient identifier identifying the patient (S402), a radiation treatment agent is prepared for delivery to the patient according to the radiation treatment plan (S403), and the radiation treatment agent is associated with the identifier (S404).

## Description

### CROSS REFERENCE TO RELATED APPLICATIONS

This application claims priority to Provisional Application Serial No. 60/529,124, filed December 12, 2003 and entitled "Modular Coordinating System for Combinatorial Products".

### BACKGROUND

### Field

The claimed invention relates generally to medical treatment using a radiation dose-enhancing agent.

### Description

According to conventional radiation treatment, a beam of radiation is directed toward a tumor located within a patient. The radiation beam delivers a predetermined dose of therapeutic radiation to the tumor according to a treatment plan. The delivered radiation kills cells of the tumor by causing ionizations within the cells.

A kilovoltage radiation treatment system produces a divergent beam of X-ray radiation having energies in the 50 to 150 keV range and focuses the beam on a target site using a lens designed for this purpose. At these energies, most cellular damage caused by the radiation beam is due to photoelectric absorption. The amount of absorption, and the resulting cellular damage, may be magnified by injecting a dose-enhancing biochemical agent into the target site. Some kilovoltage radiation treatment systems employing this technique are described in U.S. Patent Nos. 6,125,295 and 6,366,801.

The dose-enhancing effect of dose-enhancing agents can be beneficial, since cure rates for tumors often increase with increased radiation doses. A dose-enhancing agent should be delivered in prescribed amounts and according to prescribed parameters in order to ensure that particular tissues experience the radiation doses specified by an associated radiation treatment plan. Improved efficiency and/or control of such delivery are desired.

### SUMMARY

To address at least the foregoing, some embodiments of the present invention provide a system, method, apparatus, and means to generate a radiation treatment plan associated with a patient, associate the radiation treatment plan with an identifier and a patient identifier identifying the patient, prepare a radiation treatment agent for delivery to the patient according to the radiation treatment plan, and associate the radiation treatment agent with the identifier. In a further aspect, the identifier associated with the radiation treatment agent is determined, the patient identifier is determined, the radiation treatment plan associated with the identifier and the patient is determined, and the radiation treatment agent is delivered to the patient in accordance with the radiation treatment plan.

According to some embodiments, an identifier associated with a radiation treatment agent is determined, a patient identifier is determined, a radiation treatment plan associated with the identifier and the patient identifier is determined, and the radiation treatment agent is delivered to a patient in accordance with the radiation treatment plan.

In still further aspects, a system includes a radiation treatment agent to treat tissue in response to received X-ray radiation, and an identifier associated with the radiation treatment agent, wherein the identifier is usable to identify a radiation treatment plan. The system may further include a medium storing the radiation treatment plan in a computer-readable format, wherein the radiation treatment plan is not associated with a particular patient. Alternatively, the system may include a treatment planning system storing the radiation treatment plan, wherein the radiation treatment plan is associated with a patient identifier.

The claimed invention is not limited to the disclosed embodiments, however, as those in the art can readily adapt the description herein to create other embodiments and applications.

### BRIEF DESCRIPTION OF THE DRAWINGS

The construction and usage of embodiments will become readily apparent from consideration of the following specification as illustrated in the accompanying drawings, in which like reference numerals designate like parts, and wherein:
FIG. 1 is a perspective view of a system according to some embodiments;
FIG. 2 is a diagram illustrating a radiation treatment room according to some embodiments;
FIG. 3 is a block diagram illustrating elements of a radiation treatment system according to some embodiments;
FIG. 4 is a flow diagram of process steps according to some embodiments;
FIG. 5 is a representative view of a portion of a data table according to some embodiments;
FIG. 6 is a perspective view of a delivery device, a radiation treatment agent and an identifier associated with the agent according to some embodiments;
FIG. 7 is a flow diagram of process steps according to some embodiments; and

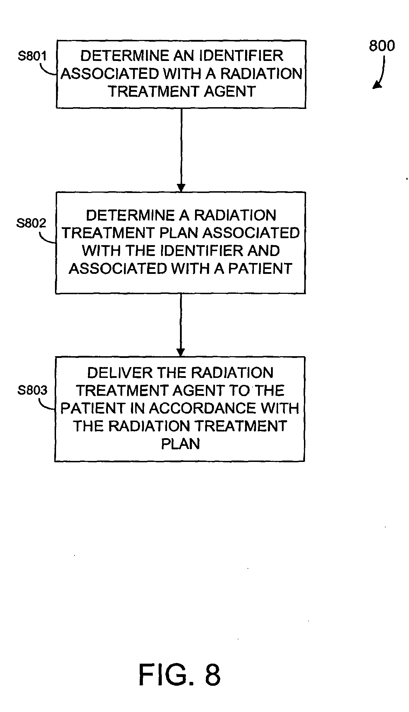

FIG. 8 is a flow diagram of process steps according to some embodiments.

### DETAILED DESCRIPTION

The construction and usage of embodiments will become readily apparent from consideration of the following specification as illustrated in the accompanying drawings, in which like reference numerals designate like parts, and wherein:

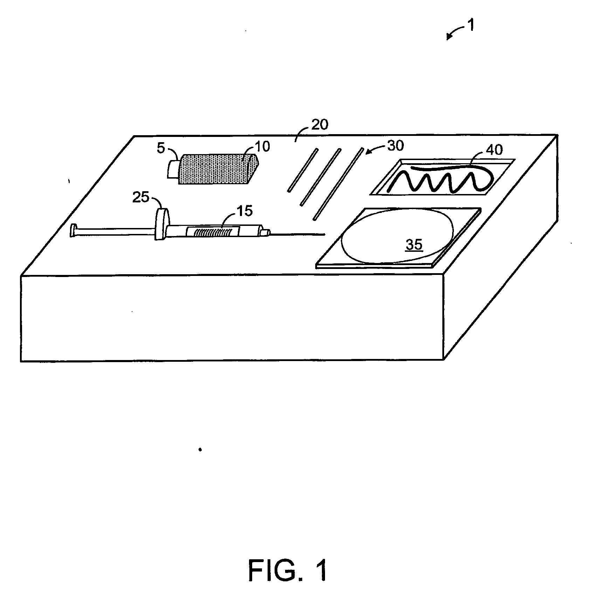

FIG. 1 is a perspective view of a system according to some embodiments. System 1 comprises container 5 containing radiation treatment agent 10, and identifier 15 associated with radiation treatment agent 10. According to some embodiments, identifier 15 is usable to identify a radiation treatment plan. As will be described in detail below, the identified radiation treatment plan might be associated with a patient identifier and/or might not be associated with any particular patient.

Radiation treatment agent 10 may comprise any currently- or hereafter-known composition that is capable of treating tissue in response to received X-ray radiation. When subjected to radiation in the kilovoltage energy range, the absorption cross-section of an element having an atomic weight greater than fifty is often significantly higher than elements of which most human tissue is composed. Therefore, if a suitable radiation beam irradiates a tissue volume containing such an element, more photons will be stopped by the volume than would be in the absence of the element. The resulting tissue damage will be greater than tissue damage that would occur without the element, because most of the increased stoppages will be due to photoelectric absorption.

Accordingly, radiation treatment agent 10 may comprise a heavy element-carrying biochemical agent in some embodiments. Agent 10 according to some embodiments carries one or more of Iodine, Gadolinium, or Gold. Leukine may be employed as radiation treatment agent 10, due to its ability to treat tissue in response to received X-ray radiation.

Container 5, agent 10, and identifier 15 are disposed within package 20. Also included in package 20 are syringe 25 on which identifier 15 is disposed, needles 30, software medium 35 and catheter 40. Syringe 25, needles 30 and catheter 40 comprise devices for delivering agent 10 to a patient. One or more of syringe 25, needles 30 and catheter 40 may be particularly suitable to the delivery of a radiation treatment agent having the composition and/or concentration of agent 10.

Software medium 35 may store a radiation treatment plan in a computer-readable format. The radiation treatment plan might not be associated with any particular patient. Software medium 35 may also or alternatively store computer-executable process steps to calculate therapeutic effects of radiation treatment agent 10. Such steps may comprise steps to model the dissipation of agent 10 within tissue, to calculate a dose enhancement due to the receipt by agent 10 of X-rays having particular energies, or any other agent-related steps.

System 1 may include less or more elements than depicted in FIG. 1. Non-exhaustive examples of such elements include one or more of patient positioning devices, assays, radiation sources, or other radiation treatment agents. System 1 may also be packaged in suitable manners other than that shown in FIG. 1.

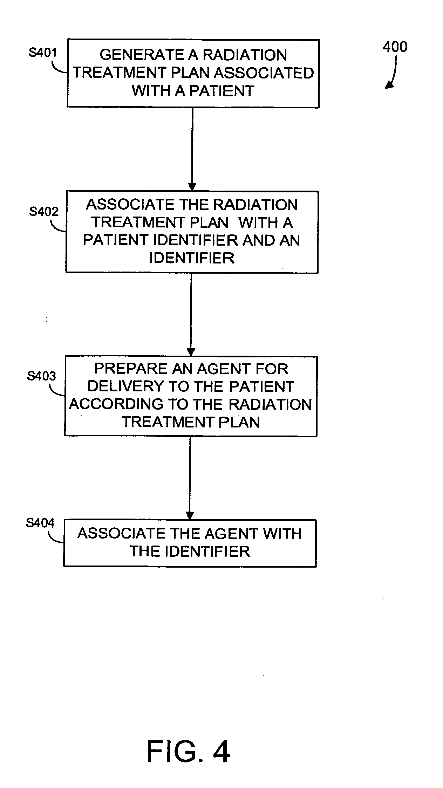

System 1 may be aggregated by an entity that is to deliver radiation treatment, such as a hospital. An example of the foregoing is provided below with respect to FIG. 4. Briefly, a radiation oncologist may generate a radiation treatment plan based on previously-acquired computed tomography scans of a patient and may associate the treatment plan with a patient identifier and identifier 15. System 1 may then be created with elements 5 through 40 that are selected particularly for execution of the radiation treatment plan. Identifier 15 may therefore be used to confirm that system 1 and radiation treatment agent 10 are associated with the radiation treatment plan.

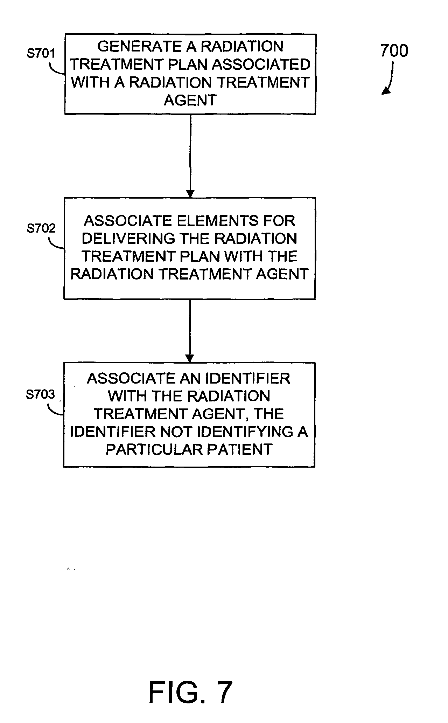

The elements of system 1 may be associated within package 20 by any one or more entities. A manufacturer or reseller of agent 10 may create system 1 and provide system 1 to entities that deliver radiation treatment. According to some of these embodiments, an example of which is described with respect to FIG. 7, software medium 35 may store a radiation treatment plan that is not associated with any particular patient. For example, the radiation treatment plan may be suitable for particular-sized tumors at particular tissue locations, and the volume and composition of agent 10 (as well as the selection of elements 25 through 40) may be suitable for treating a tumor of the certain size and location. A manufacturer or reseller may also produce system 1 in view of a particular patient as described above.

In each of the above cases, identifier 15 is associated with radiation treatment agent 10. Such an association may provide more efficient and more reliable execution of a radiation treatment plan.

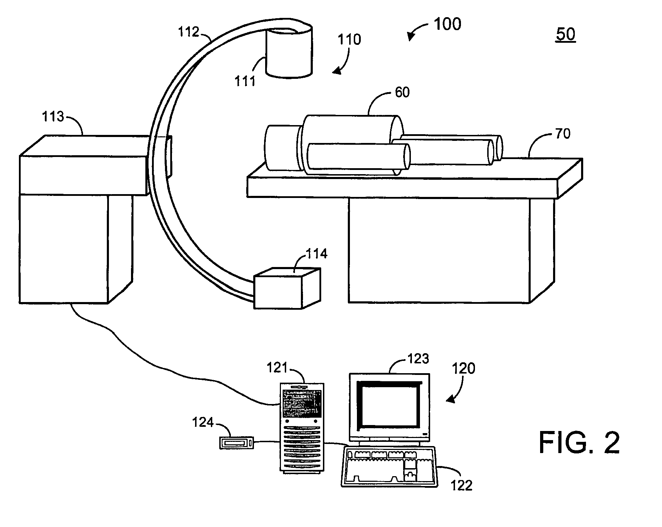

FIG. 2 illustrates radiation treatment room 50 according to some embodiments. Radiation treatment room 50 comprises patient 60, table 70 and delivery system 100. According to some embodiments, delivery system 100 is used to deliver radiation to patient 60 according to a radiation treatment plan.

Radiation unit 110 includes treatment head 111, C-arm 112, base 113 and imaging system 114. Treatment head 111 includes a beam-emitting device such as an X-ray tube for emitting radiation used during calibration, data acquisition and/or treatment. The radiation may comprise electron, photon or any other type of radiation, and may have energies ranging from 50 to 150 keV. The radiation emitted by treatment head 111 may comprise any radiation suitable for data acquisition and/or treatment according to some embodiments. In some embodiments, the radiation is suitable to produce dose-enhancing effects when used in conjunction with a radiation treatment agent that is capable of treating tissue in response to received X-ray radiation.

Treatment head 111 also includes a cylinder in which are disposed optics such as a focusing lens for optically processing the emitted radiation. The focusing lens may comprise a lens for producing a convergent radiation beam from radiation emitted by the X-ray tube. Examples of this type of lens are described in U.S. Patent No. 6,359,963 to Cash, in U.S. Patent No. 5,604,782 to Cash, Jr., in U.S. Patent Application Publication No. 2001/0043667 of Antonell et al., and/or elsewhere in currently or hereafter-known art. Treatment head 111 may also include beam-shaping devices such as one or more jaws, collimators, reticles and apertures.

Imaging device 114 may comprise an image intensifier and a camera. An image intensifier is a vacuum tube that converts X-rays to visible light, which is then detected by the camera to produce an image. Imaging device 114 may comprise a flat-panel imaging device using a scintillator layer and solid-state amorphous silicon photodiodes deployed in a two-dimensional array. The RID1640, offered by Perkin-Elmer®, Inc. of Fremont, California, is one suitable device.

Imaging device 114 may comprise other types of imaging devices. For example, X-ray radiation may also be converted to and stored as electrical charge without use of a scintillator layer. In such imaging devices, X-rays are absorbed directly by an array of amorphous selenium photoconductors. The photoconductors convert the X-rays directly to stored electrical charge that comprises an acquired image of a radiation field. Imaging device 114 may also comprise a CCD or tube-based camera. Such an imaging device may include a light-proof housing within which are disposed a scintillator, a mirror, and a camera.

Treatment head 111 and imaging device 114 may be coupled to C-arm 112 so as to face one another irrespective of any movement of C-arm 112 with respect to base 113. In this regard, C-arm 112 is slidably mounted on base 113 and can therefore be moved in order to change the position of treatment head 111 with respect to table 70. Table 70 may also be adjustable to assist in positioning an internal portion of patient 60 with respect to radiation unit 110. In some embodiments, base 113 includes a high-voltage generator for supplying power used by treatment head 111 to generate kilovoltage radiation.

Many C-arm/base configurations may be used in conjunction with some embodiments, including portable configurations, configurations in which base 113 is rotatably mounted to a ceiling of radiation treatment room 50, configurations in which one C-arm is slidably mounted on another C-arm, and configurations incorporating multiple independent C-arms. Embodiments of radiation unit 110 may comprise one of the SIREMOBIL®, MULTISTAR®, BICOR® and POLYSTAR® systems produced by Siemens Corporation® or other systems designed to emit treatment radiation.

Operator station 120 includes processor 121 in communication with keyboard 122, display 123 and identifier input device 124. An operator may operate operator station 120 to instruct radiation unit 110 to deliver X-ray radiation to patient 60 according to a radiation treatment plan stored in processor 121. Operator station 120 may also or alternatively be used to generate the radiation treatment plan. In this regard, operator station 120 may generate the treatment plan by importing computed tomography images and executing inverse treatment planning based on the images. The treatment plan may then be exported to an application for controlling radiation unit 110.

Identifier input device 124 may be used to input information such as identifier 15 and/or patient identifiers to operator station 120. Identifier input device 124 may comprise one or more of a smart card scanner, a barcode scanner, a fingerprint scanner, a keypad, or any other input device. Information input by identifier input device 124 may be used to identify a radiation treatment plan to be executed.

Operator station 120 may be located apart from radiation unit 110, such as in a different room, in order to protect the operator from radiation. It should be noted, however, that the operation of low-voltage radiation systems does not require protective measures to the extent of those taken during megavoltage radiation treatment, often resulting in less costly treatment.

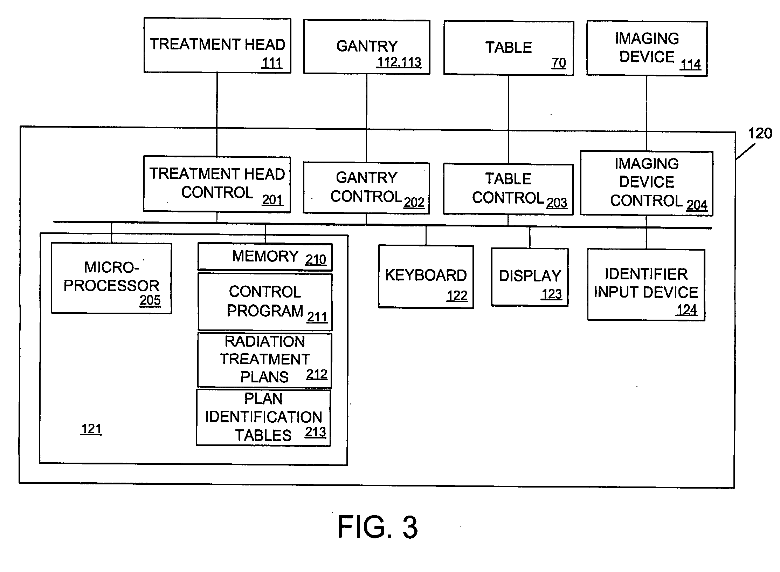

FIG. 3 is a block diagram of elements of radiation treatment room 50 according to some embodiments. As shown, operator station 120 includes several elements for interfacing with other elements of room 50. Specifically, operator station 120 includes treatment head control 201, gantry control 202, table control 203, and imaging device control 204. Processor 121 further includes microprocessor 205 and memory 210.

Treatment head control 201 controls treatment head 11 so as to implement particular radiation delivery parameters called for by a radiation treatment plan. These parameters may include an X-ray tube potential, a radiation energy, an X-ray tube current, a scan time and radiation filtration parameters. Gantry control 202, table control 203 and imaging device control 204 also operate to control C-arm 112, base 113, table 70 and imaging device 114 in accordance with a radiation treatment plan.

Microprocessor 205 executes processor-executable process steps stored in memory 210. In this regard, memory 210 stores processor-executable process steps of control program 211. Control program may comprise a software application to control elements of room 50 based on a radiation treatment plan. In this regard, memory 210 may also store radiation treatment plans 212 and plan identification table 213. Radiation treatment plans 212 may comprise scripts that are automatically executable by radiation unit 110 and treatment table 70 in order to provide multiple treatment segments. Radiation treatment plans 212 may also comprise any other currently- or hereafter-known types of radiation treatment plan.

Plan identification table 213 may associate identifiers and patient identifiers with radiation treatment plans. The identifiers may in turn be associated with radiation treatment agents. In some embodiments, plan identification table 213 may allow for identification of one of radiation treatment plans 212 based on a patient identifier and an identifier associated with a treatment agent. The structure and use of plan identification table 213 according to some embodiments will be described in detail below.

The radiation delivery environment illustrated in FIGS. 2 and 3 may include less or more elements than those shown. In addition, embodiments are not limited to the devices and/or to the illustrated environment.

FIG. 4 is a flow diagram of process steps 400 according to some embodiments. Process steps 400 may be embodied, in whole or in part, by hardware of and/or software executed by elements including but not limited to those of radiation delivery system 100. Software embodying one or more of process steps 400 may be stored by any medium, including a fixed disk, a floppy disk, a CD-ROM, a DVD-ROM, a Zip™ disk, a magnetic tape, or a signal. Some or all of such software may also be stored in one or more devices. One or more of process steps 400 may be performed manually.

Initially, at step S401, a radiation treatment plan associated with a patient is generated. The radiation treatment plan may be generated based on previously-acquired data stored in a storage device. The previously-acquired data may comprise three-dimensional data representing internal portions of the patient.

In some embodiments of step S401, a patient is placed in a computed tomography (CT) scanner to obtain CT data representing the patient using currently- or hereafter-known techniques. Such techniques may include producing sets of two-dimensional data obtained at various rotational angle positions with respect to the patient. Attenuation coefficients (Hounsfield numbers) of points within the patient are computed based on the data sets to generate three-dimensional data representing internal portions of the patient. The data represents the attenuative properties of tissues at each point of the represented portions, and may be used to generate a visual representation of the relative densities of each square of material.

A radiation oncologist may generate the radiation treatment plan at step S401 based on the three-dimensional data. The radiation treatment plan may indicate a radiation dose, a radiation target, an amount and concentration of dose-enhancing agent, a C-arm position, and/or any other currently- or hereafter-known treatment plan parameter. The radiation treatment plan may be formatted as required by a treatment planning system that will be used to execute the treatment plan.

The radiation treatment plan is associated with an identifier and with a patient identifier at S402. The patient identifier may comprise any perceptible article capable of identifying the patient of step S401. According to some embodiments, a patient identifier is an alphanumeric code that uniquely identifies the patient. The patient identifier may be created during step S401 and/or may be associated with all of the patient's medical records. The identifier will be associated with an agent for treating tissue in response to received radiation. The associated agent may be usable to execute the treatment plan generated at S401.

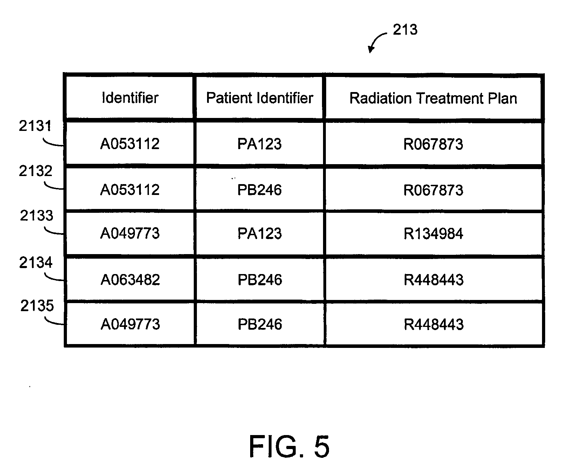

FIG. 5 illustrates a tabular representation of a portion of plan identification table 213. According to some embodiments of S402, the radiation treatment plan, the patient identifier and the identifier are associated with one another in a record of plan identification table 213. A radiation treatment plan is identified in table 213 by a code that may serve as an index to a radiation treatment plan stored among radiation treatment plans 212. In the present example, it is assumed that record 2133 is created at step S402.

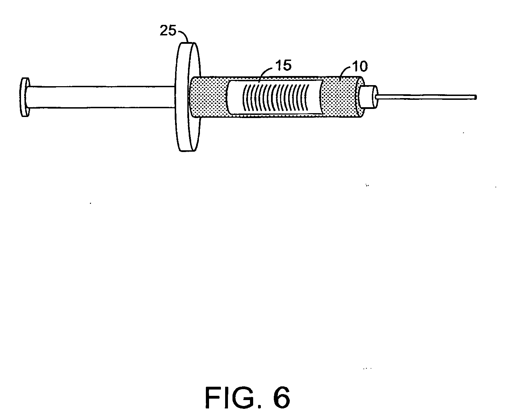

Next, at step S403, an agent is prepared for delivery to the patient according to the treatment plan generated at step S401. Preparation at step S403 may comprise measuring an appropriate amount of agent according to the treatment plan and preparing a delivery device to deliver the agent. FIG. 6 is a perspective view of agent 10 after some embodiments of step S403. Agent 10 has been measured and placed into syringe 25. Syringe 25 may be particularly suitable to the delivery of agent 10 and/or particularly suitable to delivery as required by the generated treatment plan.

The agent is associated with the identifier at step S404. Continuing with the foregoing example, barcode 15 is placed on syringe 25 according to some embodiments of step S404. Barcode 15 may encode the identifier that was mentioned with respect to step S402. Based on record 2133 of table 213, barcode 15 encodes the identifier "A49773".

Any suitable system for associating an agent with an identifier may be used at step S404. As non-exhaustive examples, the identifier may be placed on a container containing the agent such as container 5, on a package containing the agent and related elements such as package 20, or on a surgical tray holding the agent and delivery devices used to deliver the agent. The identifier itself may comprise any perceptible article capable of identifying the agent. Such articles include but are not limited to printed patterns, smells, colors, masses, and electronic identification tags.

Embodiments of process steps 400 may set the stage for more efficient and/or more reliable execution of a radiation treatment plan than previously available systems. Process steps 700 of FIG. 7 may also conform to embodiments that provide for increased reliability and/or efficiency of a subsequently-executed radiation treatment plan.

Process steps 700 may be embodied, in whole or in part, by hardware of and/or software executed by elements including but not limited to those of radiation delivery system 100. Software embodying one or more of process steps 700 may be stored by any medium, including a fixed disk, a floppy disk, a CD-ROM, a DVD-ROM, a Zip™ disk, a magnetic tape, or a signal. Some or all of such software may also be stored in one or more devices. One or more of process steps 700 may be performed manually.

Process steps 700 may be performed by a manufacturer or a reseller of a radiation treatment agent. According to some embodiments, process steps 700 are executed to produce a package that may be sold to entities that deliver radiation treatment to patients.

At step S701, a radiation treatment plan associated with a radiation treatment agent is generated. The radiation treatment plan may be generated based on characteristics of the radiation treatment agent, a particular formulation of the radiation treatment agent, a volume of the radiation treatment agent, and/or an intended use of the radiation treatment agent. For example, the radiation treatment plan may be intended to treat lung tumors using a particular radiation treatment agent.

The radiation treatment plan may therefore not be associated with a particular patient. The radiation treatment plan generated at step S701 may be incomplete and later customizable based on a particular patient to whom the plan is to be delivered. A software medium such as medium 35 may be used to store the generated radiation treatment plan.

Elements for delivering the radiation treatment plan are associated with the radiation treatment agent at step S702. The elements may be particularly called for by the radiation treatment plan. FIG. 1 illustrates one embodiment for associating the delivery elements with the radiation treatment agent. In this regard, the association at step S702 may comprise physically collecting the delivery elements with the radiation treatment agent. The elements may also be associated with the agent by placing identical indicia on each element.

Next, at step S703, an identifier is associated with the radiation treatment agent. The identifier does not identify any particular patient. According to some embodiments, step S703 comprises placing barcode 15 on syringe 25 as shown in FIG. 1. Step S703 may also comprise placing an indicia on package 20 or any other system for associating an identifier with a radiation treatment agent.

Process steps 700 may be used to create a generic kit that may be used to deliver a radiation treatment agent to any individual patient. Process steps 700 may also provide for more reliable delivery of the radiation treatment agent according to a radiation treatment plan.

Process steps 800 may be used to deliver a radiation treatment agent according to a radiation treatment plan. Process steps 800 may be embodied, in whole or in part, by hardware of and/or software executed by elements including but not limited to those of radiation delivery system 100. Software embodying one or more of process steps 800 may be stored by any medium, including a fixed disk, a floppy disk, a CD-ROM, a DVD-ROM, a Zip™ disk, a magnetic tape, or a signal. Some or all of such software may also be stored in one or more devices. One or more of process steps 800 may be performed manually.

An identifier associated with a radiation treatment agent is determined at step S801. In some embodiments, the identifier is represented by barcode 15 located on syringe 25. Scanner 124 may determine the identifier by scanning barcode 15. Any other identifier associated with a radiation treatment agent and system for determining the identifier may be used at step S801.

A radiation treatment plan is determined at step S802. The radiation treatment plan is associated with the identifier and with a patient. Prior to some embodiments of step S802, scanner 124 may read a patient identifier from a patient tag or smart card. Accordingly, the identifier and the patient identifier may be used in conjunction with plan identification table 213 to identify a radiation treatment plan from among radiation treatment plans 212.

Records 2131 and 2132 of table 213 show that a same radiation treatment plan and a same radiation treatment agent may be associated with more than one patient. Records 2131 and 2132 may represent a radiation treatment plan that was associated with a radiation treatment agent as described with respect to process steps 700. Records 2133 through 2135 show radiation treatment plans that are unique to each patient identified therein, which may indicate that these plans were generated as described with respect to process steps 400. Moreover, records 2134 and 2135 show that a same radiation treatment plan may be associated with more than one radiation treatment agent.

The radiation treatment agent is delivered to the patient in accordance with the determined treatment plan in step S803. The agent may be delivered via direct injection, intravenous injection, or by other means. In this regard, the identifier may also be associated with delivery devices to be used in the delivery of the agent.

Process steps 800 may provide for more reliable and/or efficient delivery of a radiation treatment agent in accordance with a radiation treatment plan. In some embodiments, treatment radiation is delivered according to the radiation treatment plan at the conclusion of process steps 800. The radiation treatment agent may then treat tissue of the patient in response to the received radiation.

Those in the art will appreciate that various adaptations and modifications of the above-described embodiments can be configured without departing from the scope and spirit of the claims. Therefore, it is to be understood that the claims may be practiced other than as specifically described herein.

## Claims

1. A method comprising:
generating (S401) a radiation treatment plan (212) associated with a patient (60);
associating (S402) the radiation treatment plan with an identifier (15) and a patient identifier identifying the patient;
preparing (S403) a radiation treatment agent (10) for delivery to the patient according to the radiation treatment plan; and
associating (S404) the radiation treatment agent with the identifier.

2. A method according to Claim 1, further comprising:
determining the identifier associated with the radiation treatment agent;
determining the patient identifier;
determining the radiation treatment plan associated with the identifier and the patient; and
delivering the radiation treatment agent to the patient in accordance with the radiation treatment plan.

3. A method according to Claim 1, wherein associating the radiation treatment agent with the identifier comprises:
placing a barcode indicative of the identifier on a container (5) containing the agent.

4. A method according to Claim 1, wherein associating the radiation treatment agent with the identifier comprises:
placing the agent in a container associated with the identifier.

5. A method according to Claim 1, wherein the radiation treatment agent comprises a dose-enhancing agent.

6. A method comprising:
determining (S801) an identifier (15) associated with a radiation treatment agent (10);
determining a patient identifier;
determining (S802) a radiation treatment plan associated with the identifier and the patient identifier; and
delivering (S803) the radiation treatment agent to a patient (60) in accordance with the radiation treatment plan.

7. A method according to Claim 6, wherein determining the identifier comprises scanning a barcode of a container (5) containing the agent.

8. A method comprising:
generating (S701) a radiation treatment plan (212) associated with a radiation treatment agent (10);
associating (S702) elements (1) for delivering the radiation treatment plan with the radiation treatment agent; and
associating (S703) an identifier (15) with the radiation treatment agent, the identifier not identifying a particular patient.

9. A method according to Claim 8, further comprising:
determining the identifier associated with the radiation treatment agent;
determining the radiation treatment plan associated with the identifier; and
delivering the radiation treatment agent to a patient in accordance with the radiation treatment plan.

10. A system comprising:
a radiation treatment agent (10) to treat tissue in response to received X-ray radiation; and
an identifier (15) associated with the radiation treatment agent,
wherein the identifier is usable to identify a radiation treatment plan.

11. A system according to Claim 10, further comprising:
a delivery device (100) to deliver the radiation treatment agent to a patient according to the radiation treatment plan.

12. A system according to Claim 10, further comprising:
a medium (35) storing the radiation treatment plan in a computer-readable format,
wherein the radiation treatment plan is not associated with a particular patient.

13. A system according to Claim 10, further comprising:
a medium (35) storing computer-executable process steps to calculate therapeutic effects of the radiation treatment agent.

14. A system according to Claim 10, wherein the radiation treatment plan indicates a radiation dose and a radiation target location.
